# EUROPEAN PATENT SPECIFICATION

(11) **EP 2 505 149 B1**
(45) Date of publication and mention of the grant of the patent: **19.06.2019**
(21) Application number: 12162377.1
(22) Date of filing: 30.03.2012
(51) Int. Cl.: A61B 17/12

(54) **Modifiable occlusion device**
Modifizierbare Verschlussvorrichtung
Dispositif d'occlusion modifiable

(30) Priority: 31.03.2011 US 201113076474
(43) Date of publication of application: 03.10.2012
(73) Proprietor: Codman & Shurtleff, Inc., Raynham, MA 02767 (US)
(72) Inventor: Slazas, Robert, Raynham, MA 02767 (US); Lorenzo, Juan, Raynham, MA 02767 (US); Forsythe, Peter, Raynham, MA 02767 (US)
(74) Representative: Carpmaels & Ransford LLP

(56) References cited:
- EP-A1- 1 652 494
- EP-A1- 1 652 495
- WO-A2-2005/046747
- US-A1- 2004 153 120
- US-A1- 2004 247 849
- US-A1- 2006 058 834
- US-A1- 2009 297 582

## Description

### BACKGROUND OF THE INVENTION

### 1. Field of the Invention

The invention relates to implants within body vessels and more particularly to occlusive devices including stents which are irreversibly modified based on localized pressure differentials.

### 2. Description of the Related Art

Vascular disorders and defects such as aneurysms and other arterio-venous malformations are especially difficult to treat when located near critical tissues or where ready access to a malformation is not available. Both difficulty factors apply especially to cranial aneurysms. Due to the sensitive brain tissue surrounding cranial blood vessels and the restricted access, it is very challenging and often risky to surgically treat defects of the cranial vasculature.

In the treatment of aneurysms by endovascular methods, the goal is to exclude the internal volume of the aneurysm sac from arterial blood pressure and flow. As long as the interior walls of the aneurysm are subjected to blood pressure and/or flow, there is a risk of the aneurysm rupturing.

Non-surgical treatments include vascular occlusion devices such as embolic coils deployed using catheter delivery systems. In a currently preferred procedure to treat a cranial aneurysm, the distal end of an embolic coil delivery catheter is initially inserted into non-cranial vasculature of a patient, typically through a femoral artery in the groin, and guided to a predetermined delivery site within the cranium. The aneurysm sac is then filled with embolic material that forms a solid, thrombotic mass that protect the walls from blood pressure and flow.

One inherent drawback to embolic treatments is that the aneurysm volume is permanently maintained due to the solid embolic mass implanted within them. Even after the aneurysm walls have been relieved of blood pressure and flow impingement, the walls cannot fully heal, reshape to a less distended formation, or be reincorporated back into the parent vessel wall. Also, if the size of the aneurysm created any "mass effect" type injury to the brain, the implanted embolic mass does not allow the aneurysm to shrink significantly after treatment.

When using a neck-occlusive approach to treat an aneurysm, the entrance or "neck" of the aneurysm is treated instead of the aneurysm volume itself. If the transfer of blood across the neck can be minimized, then stasis of the blood in the aneurysm volume can lead to formation of a natural thrombotic mass without the implantation of embolic materials. A natural thrombotic mass is preferable because it allows for an increased level of healing, including reduced distension of the aneurysm walls, and perhaps possible reincorporation of the aneurysm into the original parent vessel shape along the plane of the aneurysm's neck. The neck plane is an imaginary surface where the intima of the parent artery would be if not for formation of the aneurysm.

A significant challenge for many current neck-occlusive techniques is to substantially block the aneurysm neck in the parent vessel and yet not impede flow into perforator-type vessels which branch off of the parent vessel, are very small in diameter, numerous in some anatomical locations, and yet feed clinically important regions, especially within the brain. One example is the basilar artery, which has many perforator vessels feeding the pons and upper brain stem from the parent basilar artery. The use of a non-discriminatory neck occlusive device in this type of artery can unintentionally cause severe damage to the patient if the openings, known as "ostia", of the perforator vessels are blocked.

A typical basic configuration of neck-occlusive devices is a tubular, stent-like structure. These structures can be woven or wound from various fibers, laser-cut from metal, or made in various other ways. Many have interior struts or scaffolds. What most have in common is radial symmetry, meaning that they do not cover one portion, side or radial sector of the artery more or less porously than other sectors. Their symmetric construction, and therefore coverage of artery walls, is relatively homogeneous around any given transverse slice or cross-section, except where an interior strut may further reduce porosity from a micro-level perspective.

Several embodiments of an endoluminal vascular prosthesis are described in U.S. Patent No. 6,187,036 by Shaolian et al., for example, including one embodiment having fixed perfusion ports that can be aligned with diverging arteries. This prosthesis requires careful alignment of the perfusion ports with the adjacent vessels.

One example of an occlusion device directed to sealing an aneurysm while permitting flow to adjacent vessels is disclosed in U.S. Patent No. 7,156,871 by Jones et al. An expandable stent has a covering that is normally dissolvable in blood but, upon being locally activated by an activating agent, resists dissolution where activated. This device requires precise delivery of the separate activating agent.

Document EP1652495 A1 discloses an occlusive device for treatment of an aneurysm where local removal of the covering which blocks the ostium of a nearby penetrating vessel may be achieved by post-placement treatment of the area over the ostium with an activating agent which gives rise to a chemical change in the cover material, allowing it to dissolve and reopen the ostium of the said vessel.

Another type of aneurysm occlusion system is described by Bose et al. in U.S. Patent Publication No. 2007/0239261 having a plurality of pre-formed gaps or pores which allegedly expand in response to a fluid pressure differential at a side branch vessel. Various possibilities are mentioned including deflection of bendable elements such as small paddles, elastic stretching of pores, and defeating of surface tension by increased pressure differential.

It is therefore desirable to have a device which effectively occludes a neck of an aneurysm or other arterio-venous malformation in a parent vessel without blocking flow into perforator vessels communicating with the parent vessel.

### SUMMARY OF THE INVENTION

Claim 1 defines the invention and the dependent claims disclose the preferred embodiments. An object of the present invention is to provide an occlusion device which substantially blocks flow into an aneurysm in a parent vessel yet quickly adapts to a pressure differential at an ostium of a perforator vessel to allow penetrating flow into the perforator vessel.

Another object of the present invention is to provide an occlusion device which is sensitive to a differentiating characteristic between the neck of the aneurysm and the ostium of a perforator vessel.

This invention results from the realization that the neck of an aneurysm in a parent vessel can be occluded without also occluding nearby vessels, such as perforator vessels, communicating with the parent vessel by providing a device which irreversibly erodes or ruptures, including deforming, substantially only based on differential pressure and penetrating fluid flow into the perforator vessels. The device effectively senses the presence of an ostium of a perforator vessel and modifies itself to permit flow into the ostium, thereby minimizing ischemia, while continuing to substantially block flow into the aneurysm.

This invention features an occlusive device suitable for endovascular treatment of an aneurysm in a region of a parent vessel in a patient, including a structure having a fixed porosity and having dimensions suitable for insertion into vasculature of the patient to reach the region of the aneurysm in the parent vessel. The device further includes a frangible material supported by the structure which initially provides a substantial barrier to flow through the frangible material and is capable of at least one of localized rupturing and localized eroding, in the presence of a pressure differential arising at an ostium of a perforator vessel communicating with the parent vessel, within an acute time period to minimize ischemia downstream of the perforator vessel.

In some embodiments, the structure includes metallic struts and the frangible material includes a thin film formed of at least one of cellulose, alginate, urethane, polycaprolactone and polyglycolic acid. In other embodiments, the frangible material includes fibers such as electro-spun polyvinylidene fluoride fibers which are capable of parting to serve as the localized rupturing in the presence of the pressure differential.

In certain embodiments, the frangible material includes at least one biodegradable composition. In some embodiments, the structure includes a substantially non-biodegradable porous foam, such as solidified porous urethane, and the frangible material includes at least one biodegradable composition, such as polycaprolactone, interspersed through at least a portion of the porosity of the foam. In one embodiment, the frangible material is capable of responding to a pressure differential equivalent to one to fifty mm Hg and the acute time period is less than ten minutes. In some embodiments, the frangible material defines openings at least 10 microns in diameter prior to implantation in the patient and has a thickness ranging between 10 microns to 500 microns.

### BRIEF DESCRIPTION OF THE DRAWINGS AND PHOTOGRAPHS

In what follows, preferred embodiments of the invention are explained in more detail with reference to the drawings and photographs, in which:
FIG. 1 is a schematic side view of an occlusive device according to the present invention having a film overlying a support and positioned in a parent vessel below an aneurysm and above a perforator vessel;
FIG. 2 is a similar schematic side view of another occlusive device according to the present invention having electro-spun fibers overlying a support;
FIG. 3 is a similar schematic side view of yet another occlusive device according to the present invention having an erodible porous structure covering a support;
FIG. 4A is an enlarged schematic perspective, partial cross-sectional view of a portion of an alternative embodiment to the device shown in FIG. 3 having a durable porous structure;
FIG. 4B is a view of the durable porous structure of FIG. 4A after it has been impregnated with a selectively dissolving filler material; and
   PHOTOS 1-4 are scanning electron microscope images of successively smaller portions of the electro-spun fibers of the device illustrated in FIG. 2 at increasing magnifications of X15, X50, X200 and X2000, respectively.

### DETAILED DESCRIPTION OF THE PRESENTLY PREFERRED EMBODIMENTS

This invention may be accomplished by an occlusive device suitable for endovascular treatment of an aneurysm in a region of a parent vessel in a patient, with at least one type of supporting structure, such as metallic struts or porous foam, and at least one type of frangible material supported by the structure. The structure has a fixed porosity and has dimensions suitable for insertion into vasculature of the patient to reach the region of the aneurysm in the parent vessel. The frangible material initially provides a substantial barrier to flow through the frangible material and is capable of at least one of localized rupturing and localized eroding, in the presence of a pressure differential arising at an ostium of a perforator vessel communicating with the parent vessel, within an acute time period to minimize ischemia downstream of the perforator vessel.

When considering the arterial system as a non-compressible fluid piping system, the aneurysm is a dead leg which does not drain by connecting to the low-pressure, venous side of the piping system. Over short time horizons, without considering growth or contraction of the aneurysm volume, any fluid volume that transfers across the neck plane must displace an equal amount of fluid volume from the aneurysm back into the parent vessel. The result is a net-zero transference across the neck plane for the aneurysm.

A perforator vessel differs from an aneurysm since the perforator vessel does drain directly or indirectly into the low pressure side of the piping system. There is a net-positive transference across the ostial plane because a given amount of fluid volume that crosses its ostial plane, that is, enters the perforator vessel through its ostium, is lost from the high pressure side of the system and does not force an equal amount back into the parent vessel as the aneurysm does.

In such a non-compressible fluid system, a net-zero transference across the neck plane causes a zero differential pressure across the neck plane. By comparison, a net-positive transference across the ostial plane can be detected by a positive differential pressure across the ostial plane. Therefore, differential pressure is a characteristic which a device can use to distinguish between the neck of an aneurysm and the ostia of perforator vessels. Since stent-like neck occlusion devices cover both a neck plane and an ostial plane in the same manner, the inventors have recognized that neck occlusion devices are needed that change their flow-impeding properties according to the presence of differential pressure across their walls, from interior to exterior.

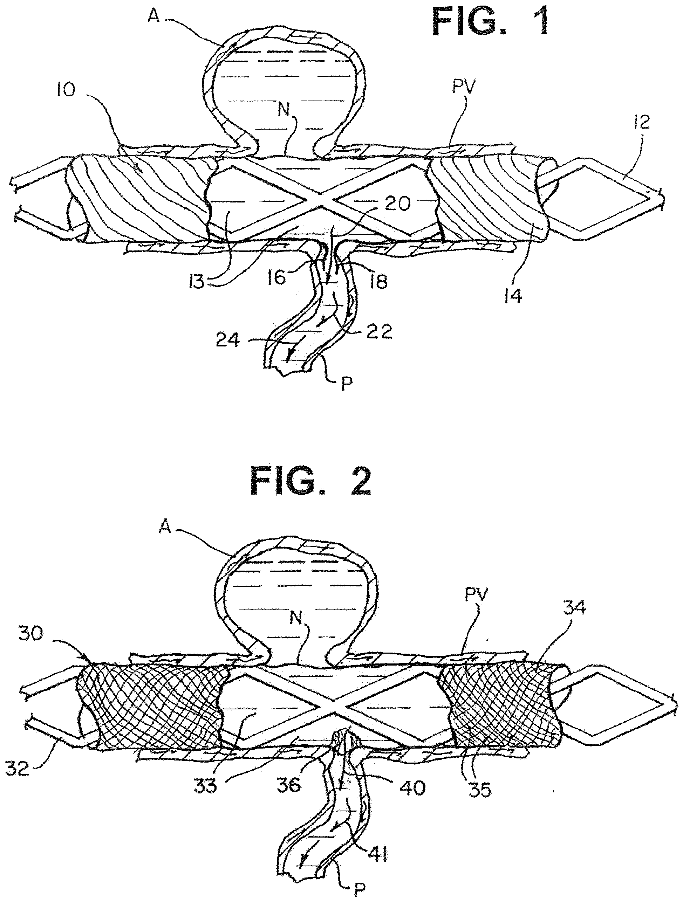

FIG. 1 schematically illustrates a tubular, stent-like device 10 according to the present invention implanted in a parent vessel PV with an upper aneurysm A and a lower perforator vessel P. Device 10 is substantially tubular and has structure such as metallic struts 12 defining relatively large openings 13 and supporting a frangible cover material 14 which includes a film-like substance that is capable of rupturing wherever a preselected differential pressure is achieved. Frangible material 14 is shown intact along the entire exterior of struts 12, including across aneurysm neck N, except where ruptured by differential pressure with resulting film flaps 16 and 18 slightly extending into the ostium of perforator vessel P. Penetrating fluid flow from parent vessel PV into perforator vessel P is illustrated by arrows 20, 22 and 24.

The frangible cover material 14 disrupts flow which would otherwise occur into aneurysm A and thereby enables a thrombus to form within aneurysm A. At the same time, frangible cover material 14 also enables blood to flow into perforator vessel P to continue feeding downstream tissues supplied by that vessel to minimize ischemia within those downstream tissues. Preferably, frangible cover material 14 provides a flow barrier at neck N for at least eight-to-twelve weeks to allow endothelial growth over device 10.

Device 10 can be either self-expanding or balloon expanded, with supporting scaffold-like structure 12 made by any of several typical stent fabrication methods. The struts 12 themselves are solid, typically metal, and do not change behavior according to the distinguishing feature of differential pressure across either an aneurysm neck or the ostium of a branching vessel. In the preferred embodiment, the struts 12 serve as a self-expanding scaffold made by laser-cutting a pattern of struts into a nitinol (NiTi) tube. The primary purposes of this structural component are to facilitate delivery of a film or other frangible cover material 14 to the target vessel, and to hold cover material 14 in apposition to the vessel wall once deployed. If the covering 14 is structurally sufficient to enable delivery and to hold position in the artery on its own, this scaffold 12 may not be needed.

The open areas 13 within the scaffold 12 are subsequently covered by a film 14 which does respond according to the level of differential pressure felt across its wall thickness. There is a net positive differential pressure across a branching vessel's ostium and none across the neck of an aneurysm, typically ranging from one to fifty mm Hg. This film 14 can be made from any number of substances, as long as it has the minimum characteristics of biocompatibility and frangibility in the presence of a preselected, sufficient differential pressure. Suitable biocompatible compositions for frangible material 14 include films or matrices of cellulose, alginate, cross-linked gels, and very thin polymer films of materials such as urethane and/or poly-glycolic acid. The film 14 need not be erodible or bioabsorbable since it is the action of rupture in the presence of sufficient differential pressure that creates the permanent, localized modification of increased flow across its wall-thickness. Similarly, although microscopic pores or other openings could be formed in the film 14 having average diameters such as described for other embodiments below, it is acceptable for the film 14 to be a continuous sheet of material because the action of rupture increases flow where needed, as sensed by sufficient differential pressure to cause the rupture.

The thickness of the film layer is determined by its desired rupture strength, but should not occupy a significant amount of cross-sectional area in the artery in order to minimize interference with normal fluid flow through the parent vessel. Less than five percent area occupation is desired. The thickness of the film is selected to achieve a desired frangibility at a minimum differential pressure within an acute time period to minimize ischemia downstream of the perforator vessel. In some constructions, the acute time period is preferably within a period of less than ten minutes, more preferably less than five minutes, in a majority of patients under typical conditions, that is, not including hypothermic or artificially depressed blood pressure conditions. The rupture strength should be adjusted so that the film is strong enough to survive delivery and placement within the target artery, but weak enough to rupture in the presence of the persistent, net-positive differential pressure across the ostium of small branching vessels. Desirable rupture strengths are expected to be in the range of 1 to 50 mmHg differential pressure.

An alternative tubular device 30, FIG. 2, according to the present invention has struts 32 which are similar to struts 12, FIG. 1, and define relatively large openings 33, FIG. 2. Device 30 further includes frangible material 34 which is formed from very thin fibers 35 in this construction that establish a porous mesh or matte outer layer. Frangible material 34 has a density sufficient to disrupt normal fluid flow at neck N to create stasis within aneurysm A to enable thrombi to form therein, yet a sufficient number of the fibers 35 part or separate to form opening 36 at the ostium of perforator vessel P when a threshold pressure differential is exceeded to enable blood to flow as illustrated by arrows 40 and 41.

In a preferred construction, these fibers 35 are applied via "electro-spinning", where a liquefied polymer such as polyvinylidene fluoride (PVDF) exiting a dispenser tip has a voltage applied to it, producing a very fine strand having an average strand thickness or diameter of one nanometer up to about ten microns. A number of controls over the construction of the fiber layer can be manipulated, such as the thickness of individual strands, the total number of strands applied, the angle at which the strand lays on the tubular scaffold, and the angles between strands which cross each other. Various electro-spinning techniques can be utilized, such as those described by Norton in U.S. Patent No. 2,048,651. Other electro-spinning techniques are described by Cooley in U.S. Patent No. 692,631, by Morton in U.S. Patent No. 705,691, and by Formhals in U.S. Patent Nos. 1,975,504 and 2,349,950 for example. The resulting characteristics of the fiber layer as manufactured, before implantation, include percentage area covered, average pore or opening size, total wall thickness, and hydraulic permeability, which provides a gross measurement of the volumetric flow rate of a certain liquid across the layer, in this case blood. In some constructions, the overall layer thickness of material 34 is about 10 microns to about 500 microns, more preferably 30 microns to 200 microns. The average opening diameter between fibers, as measured from scanning electron microscope images along a plane substantially parallel to the surface of material 35, is preferably at least 10 microns before implantation in a patient. Average openings of about 10 microns permit a small quantity of whole blood, including red blood cells, to pass through the sidewalls of device 30 to provide some nourishment to surrounding tissues, while initially providing a substantial barrier to flow through material 34. As one or more fibers rupture in the presence of sufficient differential pressure such as at the ostium of the perforator vessel P, opening 36 is preferably formed to be from 50 to 500 microns, more typically 100 to 300 microns in diameter.

One construction of device 30 is shown in PHOTOS 1-4 as scanning electron microscope images of successively smaller portions of the electro-spun fibers of device 30 at increasing magnifications of X15, X50, X200 and X2000, respectively. The left-hand side of PHOTO 1 shows fibers removed to expose the metallic struts which underlie and support the fibers, the struts defining large openings greater than one mm in this construction. A horizontal white bar illustrates a length of one mm to provide an indication of scale.

PHOTO 2 is an enlargement of the outer fiber mat layer approximately in the center of PHOTO 1. A short horizontal white bar shows a length of 100 microns. PHOTO 3 is a further enlargement showing a longer white bar also having a length of 100 microns and revealing the three-dimensional nature of the fiber mat. PHOTO 4 clearly shows the porosity of the fiber mat, with a horizontal white bar of 10 microns for scale.

The mechanism by which a sufficient number of these fibers "part" or separate in the presence of sufficient differential pressure is primarily that individual fibers will break, that is, rupture, in the localized areas of higher fluid flow. In alternate constructions, a mixture of biologically durable and degradable materials are utilized for the fibers. In regions of the fiber mesh that cover the ostium of a branching vessel, the local differential pressure is net positive and causes a persistent flow through the wall thickness of the layer. These broken fibers in the region of the layer covering the ostium of a branching vessel serve to increase the blood flow to that branching vessel preferentially compared to the region covering the aneurysm neck. The controllable factors in the construction of the frangible fiber layer 34, FIG. 2, should be adjusted such that the fibers 35 break in areas with differential pressure preselected to be a threshold rupture pressure between 1 and 50 mmHg. The thickness of the fiber layer is determined by its rupture strength, but should not occupy a significant amount of cross-sectional area in the artery. Less than five percent area occupation is desired. In some constructions, a sufficient number of fibers break or erode within an acute time period, to minimize ischemia downstream of the perforator vessel, that is preferably within a period of less than ten minutes, more preferably less than five minutes, in a majority of patients under typical conditions, that is, not including hypothermic or artificially depressed blood pressure conditions.

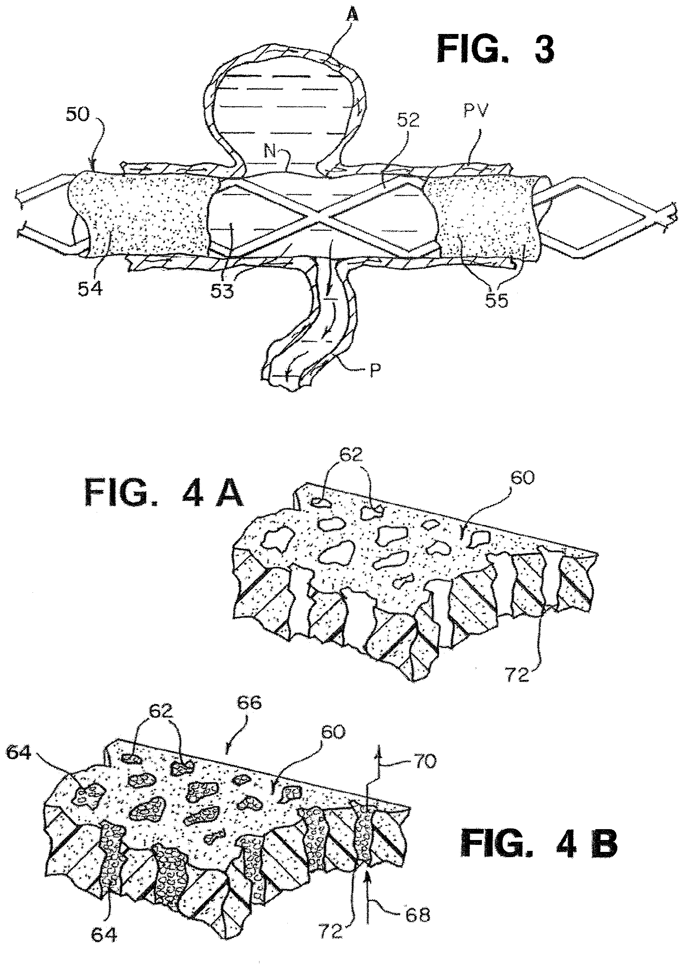

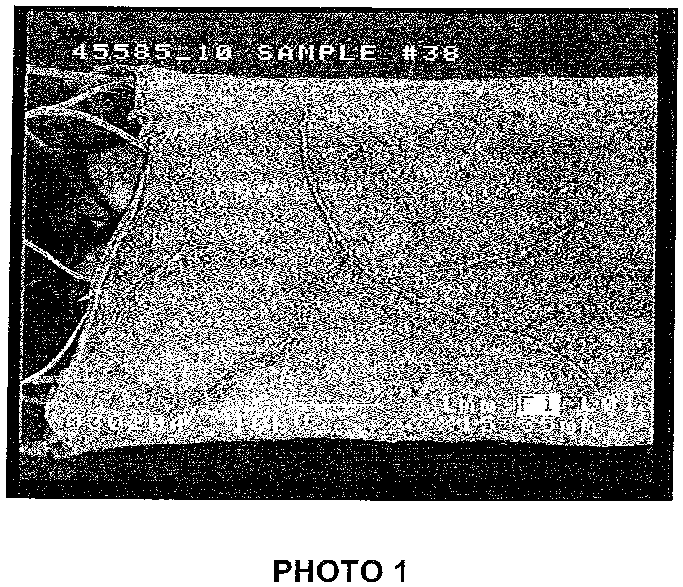

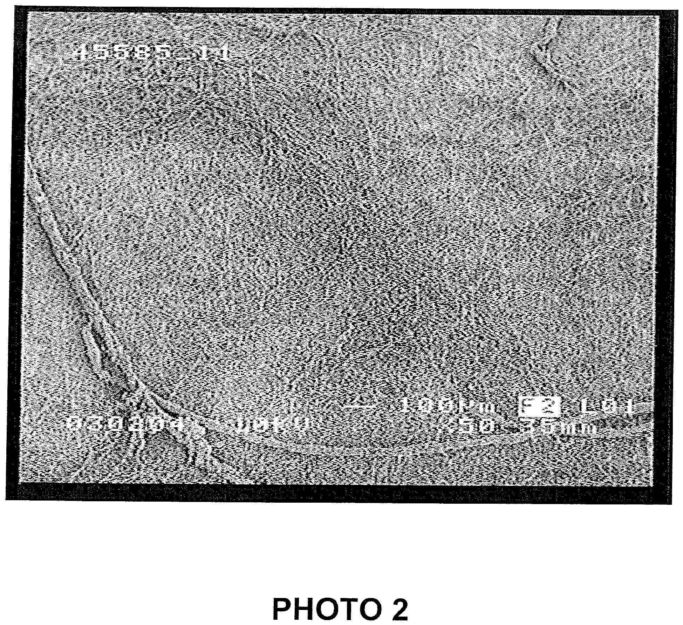

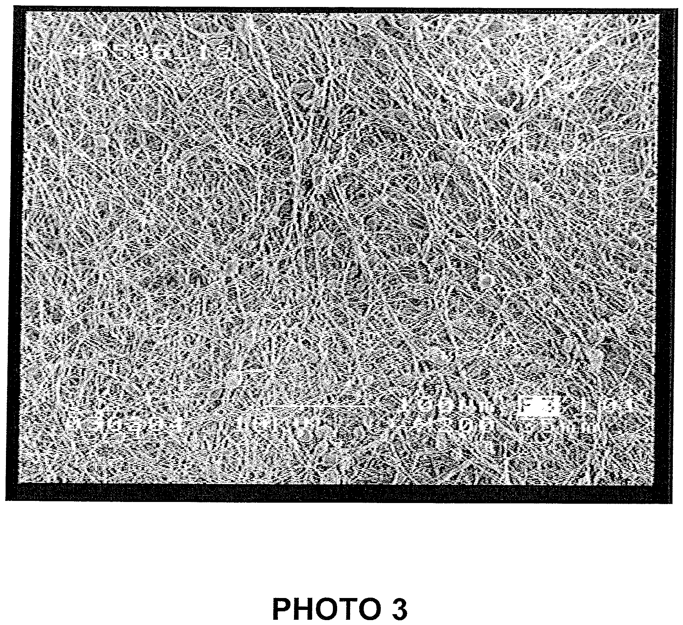

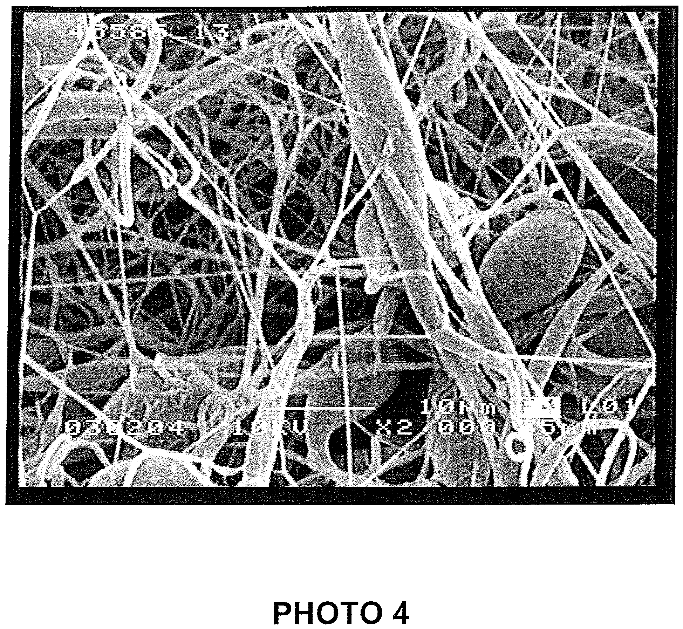

Tubular device 50, FIG. 3, is yet another embodiment of the present invention constructed with struts 52 arranged as a scaffold to define open areas or cells 53. This scaffold 52 can be either self-expanding or balloon expanded, made by any of several typical fabrication methods. The scaffold 52 is then covered with a layer 54 that has very fine pores 55 and allows a limited amount of flow across its wall thickness in the presence of a net positive differential pressure. This layer 54 can be constructed by many methods, for example foaming, lyophilization, gaseous extraction, etching, firing, or deposition. The material of layer 54 can be any biocompatible material that is subject to erosion due to fluid flow and/or erosion due to bioabsorption including consumption by live cells. In the preferred embodiment, polycaprolactone (PCL) is deposited in a somewhat sparse matrix such that it is porous as a bulk material. Other potential materials include polylactic acid (PLA), polyglycolic acid (PGA), polysaccharides, colloidal compounds, and some lipid products.

In an alternate configuration as shown in FIGS. 4A and 4B, a structure 60 of a durable, non-erodible, non-bioabsorbable material is first constructed. This flexible, elastic structure, such as a solidified urethane foam or expanded polytetrafluoroethylene (PTFE), has relatively large pores 62 so that structure 60, by itself, covers too little of the open area, has too large an average pore size, and has a hydraulic permeability that is too great to sufficiently impede or restrict flow into an aneurysm. In other words, structure 60, which may be reinforced with metal struts, establishes a maximum porosity for a device according to the present invention. Although pores 62 are shown in cross-section with relatively straight passages, such as passage 72, for simplicity of illustration, in many constructions the passages are more complex and convoluted. Pores 62 are preferably formed to be from 50 to 500 microns in average diameter, more typically 100 to 300 microns in average diameter, as measured from scanning electron microscope images along a plane substantially parallel to the surface of structure material 60.

After fabricating the structure 60, a second substance 64 that is erodible is interstitially combined with the structure 60 to form a device 66, FIG. 4B. The second material 64, such as PCL or other materials listed above, preferably is deposited as particles or a microporous foam such that the material 64 has a desired level of porosity itself, that is, it is not an impermeable bulk material. In certain constructions, material 64 defines openings having an average diameter of preferably at least 10 microns before implantation in a patient. Average openings of about 10 microns permit a small quantity of whole blood, including red blood cells, to pass through the sidewalls of device 66, as indicated by internal flow arrow 68 entering into passage 72 and external flow arrow 70 emerging from passage 72, to provide some nourishment to surrounding tissues, while initially providing a substantial barrier to flow through device 66. In the areas of net positive differential pressure, over the ostia of branching vessels, the persistent, penetrating flow through the wall of the combined layer will cause the second material 64 to respond by preferentially eroding, typically including biodegrading, more rapidly in one or more pores 62. The first purpose of the structure material 60 is to impose an upper limit on the increase in porosity, and therefore flow, to that of the structure 60 itself after all of the second material 64 has been removed. Its second purpose is to intensify the erosion, typically including biodegradation, of the second material 64 by concentrating the differential pressure provided by the branching vessel into a smaller porous area. This will improve the preferential nature by which the combined layer of device 66 will erode above branching vessels more quickly than in the general body of the device, including above an aneurysm neck.

## Claims

1. An occlusive device (10, 30, 50) suitable for endovascular treatment of an aneurysm in a region of a parent vessel in a patient, comprising:
a structure (12, 32, 52, 60) having a fixed porosity and having dimensions suitable for insertion into vasculature of the patient to reach the region of the aneurysm in the parent vessel; and
a material (14, 34, 54, 64) supported by the structure;
**characterised in that** the material is a frangiable material which initially provides a substantial barrier to flow through the frangible material and is capable of at least one of localized rupturing and localized eroding, in the presence of a pressure differential arising at an ostium of a perforator vessel communicating with the parent vessel, within an acute time period to minimize ischemia downstream of the perforator vessel.

2. The occlusive device of claim 1 wherein the structure includes metallic struts (12).

3. The occlusive device of claim 1 wherein the frangible material includes a thin film.

4. The occlusive device of claim 3 wherein the film is formed of at least one of cellulose, alginate, urethane, polycaprolactone and polyglycolic acid.

5. The occlusive device of claim 1 wherein the frangible material includes fibers (35) which are capable of parting to serve as the localized rupturing in the presence of the pressure differential.

6. The occlusive device of claim 1 or claim 5 wherein at least a substantial amount of the surface area of the frangible material defines openings at least 10 microns in diameter prior to implantation in the patient.

7. The occlusive device of claim 1 or claim 5 wherein the frangible material has a thickness ranging between 10 microns to 500 microns prior to implantation in the patient.

8. The occlusive device of claim 5 wherein the fibers (35) include electro-spun polyvinylidene fluoride fibers.

9. The occlusive device of claim 1 wherein the frangible material includes at least one biodegradable composition.

10. The occlusive device of claim 1 wherein the structure (60) includes a porous foam.

11. The occlusive device of claim 1 or claim 10 wherein the frangible material includes at least one biodegradable composition interspersed through at least a portion of the porosity of the structure/foam.

12. The occlusive device of claim 10 wherein the foam includes porous urethane.

13. The occlusive device of claim 12 wherein the biodegradable material includes polycaprolactone.

14. The occlusive device of claim 1 wherein the frangible material (14, 34, 54, 64) is capable of responding to a pressure differential equivalent to one to fifty mm Hg.

15. The occlusive device of claim 1 wherein the acute time period is less than ten minutes.

16. The occlusive device of claim 11 when dependent upon claim 1 wherein at least a substantial amount of the surface area of the biodegradable material defines openings (62) at least 10 microns in diameter prior to implantation in the patient.

17. The occlusive device of claim 11 when dependent on claim 1 wherein the structure (60) includes a substantially non-biodegradable porous foam defining the fixed porosity through which the biodegradable material is dispersed.

18. The occlusive device of claim 17 wherein the foam has a thickness ranging between 10 microns to 500 microns prior to implantation in the patient.

19. The occlusive device of claim 17 wherein the fixed porosity of the foam defines pores having an average diameter ranging between 50 microns to 500 microns.

## Patentansprüche

1. Verschlussvorrichtung (10, 30, 50), geeignet zur endovaskulären Behandlung eines Aneurysmas in einer Region eines Elterngefäßes in einem Patienten, die Folgendes umfasst:
eine Struktur (12, 32, 52, 60), die eine feste Porosität aufweist und Abmessungen aufweist, die geeignet sind zum Einsetzen in eine Vaskulatur des Patienten, um die Region des Aneurymas in dem Elterngefäß zu erreichen; und
ein Material (14, 34, 54, 64), das von der Struktur gestützt wird;
**dadurch gekennzeichnet, dass** das Material ein brüchiges Material ist, welches anfangs eine erhebliche Barriere für einen Fluss durch das brüchige Material darstellt und zum lokalisierten Reißen und lokalisierten Erodieren in der Lage ist bei Vorhandensein einer Druckdifferenz, die an einem Ostium eines Perforatorgefäßes entsteht, das mit dem Elterngefäß in Verbindung steht, innerhalb eines Akutzeitraums, um stromabwärts von dem Perforatorgefäß Ischämie zu minimieren.

2. Verschlussvorrichtung nach Anspruch 1, wobei die Struktur metallische Streben (12) beinhaltet.

3. Verschlussvorrichtung nach Anspruch 1, wobei das brüchige Material einen dünnen Film beinhaltet.

4. Verschlussvorrichtung nach Anspruch 3, wobei der Film aus Zellulose und/oder Alginat und/oder Urethan und/oder Polycaprolakton und/oder Polyglykolsäure ausgebildet ist.

5. Verschlussvorrichtung nach Anspruch 1, wobei das brüchige Material Fasern (35) beinhaltet, die zum Trennen in der Lage sind, um bei Vorhandensein der Druckdifferenz als das lokalisierte Aufreißen zu dienen.

6. Verschlussvorrichtung nach Anspruch 1 oder Anspruch 5, wobei zumindest ein erheblicher Anteil des Oberflächengebiets des brüchigen Materials vor der Implantierung in den Patienten Öffnungen von mindestens 10 Mikrometer im Durchmesser definiert.

7. Verschlussvorrichtung nach Anspruch 1 oder Anspruch 5, wobei das brüchige Material vor der Implantierung in den Patienten eine Dicke aufweist, die zwischen 10 Mikrometer und 500 Mikrometer liegt.

8. Verschlussvorrichtung nach Anspruch 5, wobei die Fasern (35) elektrogesponnene Polyvinylidenfluorid-Fasern beinhalten.

9. Verschlussvorrichtung nach Anspruch 1, wobei das brüchige Material zumindest eine biologisch abbaubare Zusammensetzung aufweist.

10. Verschlussvorrichtung nach Anspruch 1, wobei die Struktur (60) einen porösen Schaum umfasst.

11. Verschlussvorrichtung nach Anspruch 1 oder Anspruch 10, wobei das brüchige Material zumindest eine biologisch abbaubare Zusammensetzung aufweist, die durch mindestens einen Teil der Porosität der Struktur/des Schaums hindurch eingestreut ist.

12. Verschlussvorrichtung nach Anspruch 10, wobei der Schaum poröses Urethan umfasst.

13. Verschlussvorrichtung nach Anspruch 12, wobei das biologisch abbaubare Material Polycaprolakton umfasst.

14. Verschlussvorrichtung nach Anspruch 1, wobei das brüchige Material (14, 34, 54, 64) dazu in der Lage ist, auf eine Druckdifferenz, die zu einem bis fünfzig mm Hg äquivalent ist, zu reagieren.

15. Verschlussvorrichtung nach Anspruch 1, wobei der Akutzeitraum weniger als 10 Minuten beträgt.

16. Verschlussvorrichtung nach Anspruch 11, wenn von Anspruch 1 abhängig, wobei zumindest ein erheblicher Anteil des Oberflächengebiets des biologisch abbaubaren Materials vor der Implantierung in den Patienten Öffnungen (62) von mindestens 10 Mikrometer im Durchmesser definiert.

17. Verschlussvorrichtung nach Anspruch 11, wenn von Anspruch 1 abhängig, wobei die Struktur (60) einen im Wesentlichen nicht biologisch abbaubaren porösen Schaum beinhaltet, der die feste Porosität definiert, durch welche das biologisch abbaubare Material eingestreut ist.

18. Verschlussvorrichtung nach Anspruch 17, wobei der Schaum vor der Implantierung in den Patienten eine Dicke aufweist, die zwischen 10 Mikrometer und 500 Mikrometer liegt.

19. Verschlussvorrichtung nach Anspruch 17, wobei die feste Porosität des Schaums Poren definiert, die einen mittleren Durchmesser aufweisen, der zwischen 50 Mikrometer und 500 Mikrometer liegt.

## Revendications

1. Dispositif d'occlusion (10, 30, 50) approprié pour le traitement endovasculaire d'un anévrisme dans une zone d'un vaisseau parent chez un patient, comprenant :
une structure (12, 32, 52, 60) ayant une porosité fixée et ayant des dimensions appropriées pour l'insertion dans le système vasculaire du patient pour atteindre la zone de l'anévrisme dans le vaisseau parent ; et
un matériau (14, 34, 54, 64) supporté par la structure ;
**caractérisé en ce que** le matériau est un matériau cassant qui produit initialement une barrière importante à l'écoulement à travers le matériau cassant et qui peut présenter une rupture localisée et/ou une érosion localisée, en présence d'une différence de pression survenant au niveau d'un ostium d'un vaisseau perforant communicant avec le vaisseau parent, sur une très courte durée pour réduire au minimum l'ischémie en aval du vaisseau perforant.

2. Dispositif d'occlusion selon la revendication 1 dans lequel la structure comprend des entretoises métalliques (12) .

3. Dispositif d'occlusion selon la revendication 1 dans lequel le matériau cassant comprend un film mince.

4. Dispositif d'occlusion selon la revendication 3 dans lequel le film est constitué de cellulose, d'alginate, d'uréthane, de polycaprolactone et/ou de poly(acide glycolique).

5. Dispositif d'occlusion selon la revendication 1 dans lequel le matériau cassant comprend des fibres (35) qui peuvent se séparer pour servir de rupture localisée en présence de la différence de pression.

6. Dispositif d'occlusion selon la revendication 1 ou la revendication 5 dans lequel au moins une quantité importante de la surface du matériau cassant délimite des ouvertures d'au moins 10 micromètres de diamètre avant implantation chez le patient.

7. Dispositif d'occlusion selon la revendication 1 ou la revendication 5 dans lequel le matériau cassant a une épaisseur allant de 10 micromètres à 500 micromètres avant implantation chez le patient.

8. Dispositif d'occlusion selon la revendication 5 dans lequel les fibres (35) comprennent des fibres de poly(fluorure de vinylidène) électrofilées.

9. Dispositif d'occlusion selon la revendication 1 dans lequel le matériau cassant comprend au moins une composition biodégradable.

10. Dispositif d'occlusion selon la revendication 1 dans lequel la structure (60) comprend une mousse poreuse.

11. Dispositif d'occlusion selon la revendication 1 ou la revendication 10 dans lequel le matériau cassant comprend au moins une composition biodégradable intercalée dans au moins une partie de la porosité de la structure/mousse.

12. Dispositif d'occlusion selon la revendication 10 dans lequel la mousse comprend de l'uréthane poreux.

13. Dispositif d'occlusion selon la revendication 12 dans lequel la matière biodégradable comprend de la polycaprolactone.

14. Dispositif d'occlusion selon la revendication 1 dans lequel le matériau cassant (14, 34, 54, 64) peut répondre à une différence de pression équivalente à un à cinquante mm de Hg.

15. Dispositif d'occlusion selon la revendication 1 dans lequel la très courte durée est inférieure à dix minutes.

16. Dispositif d'occlusion selon la revendication 11 lorsqu'elle dépend de la revendication 1 dans lequel au moins une partie importante de la surface de la matière biodégradable délimite des ouvertures (62) d'au moins 10 micromètres de diamètre avant implantation chez le patient.

17. Dispositif d'occlusion selon la revendication 11 lorsqu'elle dépend de la revendication 1 dans lequel la structure (60) comprend une mousse poreuse pratiquement non biodégradable délimitant la porosité fixée dans laquelle la matière biodégradable est dispersée.

18. Dispositif d'occlusion selon la revendication 17 dans lequel la mousse a une épaisseur allant de 10 micromètres à 500 micromètres avant implantation chez le patient.

19. Dispositif d'occlusion selon la revendication 17 dans lequel la porosité fixée de la mousse délimite des pores ayant un diamètre moyen allant de 50 micromètres à 500 micromètres.
